# FASCICULE DE BREVET EUROPEEN

(11) **EP 1 005 323 B1**
(45) Date de publication et mention de la délivrance du brevet: **14.11.2001**
(21) Numéro de dépôt: 98942750.5
(22) Date de dépôt: 18.08.1998
(51) Int. Cl.: A61K 7/06, A61K 7/00, A61K 7/48

(54) **COMPOSITION COSMETIQUE CONTENANT UNE DISPERSION AQUEUSE DE POLYMERE ET UNE EMULSION DE SILICONE DISILANOL ET PROCEDE**
KOSMETISCHE ZUSAMMENSETZUNG DIE EINE WÄSSRIGE POLYMERDISPERSION UND EINE EMULSION EINES DISILANOLSILIKONS ENTHÄLT UND VERFAHREN
COSMETIC COMPOSITION CONTAINING A POLYMER AQUEOUS DISPERSION AND A DISILANOL SILICONE EMULSION AND METHOD

(30) Priorité: 19.08.1997 FR 9710482
(43) Date de publication de la demande: 07.06.2000
(73) Titulaire: L'OREAL, 75008 Paris (FR)
(72) Inventeur: DUPUIS, Christine, F-75018 Paris (FR); CONDAMINE, Christiane, F-95610 Eragny S/Oise (FR)
(74) Mandataire: Bourdeau, Françoise
(86) Numéro de dépôt international: FR9801817
(87) Numéro de publication internationale: WO9908652

(56) Documents cités:
- EP-A- 0 240 349
- EP-A- 0 410 899
- EP-A- 0 758 545
- WO-A-92/21316

## Description

L'invention concerne une composition cosmétique aqueuse ou hydroalcoolique contenant une dispersion aqueuse de particules insolubles de polymère filmogène et une émulsion de silicone α,ω-disilanol non aminée. L'invention concerne également un procédé de traitement cosmétique des matières kératiniques à l'aide de ces compositions.

Depuis quelques années, un intérêt tout particulier s'est manifesté pour la réalisation de compositions cosmétiques capillaires essentiellement aqueuses. En effet, l'emploi d'alcool tel que l'éthanol ou l'isopropanol, seul ou en mélange avec une faible proportion d'eau, peut présenter certains inconvénients, notamment une augmentation de l'inflammabilité lorsque la composition est sous forme d'une laque aérosol.

De manière encore plus générale, on cherche à réduire l'emploi des composés volatils à la pression atmosphérique, dits COV (Composés Organiques Volatils), qui sont présents dans les compositions cosmétiques. Les COV sont principalement les propulseurs et certains solvants tels que l'éthanol.

Pour diminuer la quantité de COV, on a essayé de remplacer les solvants tels que l'éthanol par de l'eau. Toutefois, si la plupart des polymères filmogènes hydrosolubles peuvent, en solution dans l'eau, conduire à l'obtention de compositions de fixation des cheveux, ces dernières présentent des inconvénients majeurs. Ainsi, les compositions essentiellement aqueuses de ces polymères ne permettent pas d'obtenir de hauts degrés de fixation. Il a certes été proposé d'utiliser ces polymères hydrosolubles à des concentrations élevées, mais l'augmentation de concentration provoque un tel accroissement de la viscosité des compositions que l'on ne peut que très difficilement obtenir une pulvérisation satisfaisante. Même si une pulvérisation correcte est obtenue, ces compositions aqueuses présentent un temps de séchage particulièrement long par rapport aux compositions alcooliques, et sont donc d'un faible intérêt pratique. WO92/21316 divulgue des compositions capillaires comprenant silicone α,ω-disilanols.

Il a également été proposé d'utiliser des dispersions aqueuses de particules insolubles de polymères à la place des polymères solubilisés dans des compositions aqueuses, alcooliques ou hydroalcooliques.

Cependant, jusqu'à présent, les résultats obtenus ne sont pas encore satisfaisants. En effet, si le pouvoir fixant est suffisant et le temps de séchage est acceptable, les propriétés cosmétiques ne sont pas encore satisfaisantes. En particulier, les propriétés de démêlage, de douceur et de toucher ne sont pas satisfaisantes. De plus l'élimination du polymère lors du lavage des cheveux avec un shampooing est difficile.

On a déjà essayé d'améliorer les propriétés cosmétiques des compositions cosmétiques contenant une dispersion de polymère sans diminuer le pouvoir fixant en ajoutant une silicone polyoxyalkylénée ou huile de silicone non fonctionnalisée, mais la demanderesse a constaté que contrairement à ce qui était attendu, les propriétés telles que le démêlage, la douceur ou le toucher ne sont pas améliorés et sont même dans certains cas dégradées.

La demanderesse a maintenant découvert qu'une composition cosmétique contenant, dans un milieu cosmétiquement acceptable, une dispersion aqueuse de particules de polymères insolubles et une émulsion de silicone α,ω-disilanol non aminée, permettait de remédier aux inconvénients décrits ci-dessus.

Ces compositions présentent donc un bon pouvoir fixant et de bonnes propriétés cosmétiques telles que le démêlage, la douceur, le toucher. Le coiffage ou le brossage des cheveux après application est facile.

Les compositions selon l'invention permettent d'obtenir une bonne pulvérisation, le spray est régulier et les gouttes pulvérisées sont fines. Les compositions se répartissent facilement sur l'ensemble de la chevelure. De plus, de façon surprenante, le pouvoir fixant des compositions n'est pas diminué par l'addition d'une silicone. Enfin, les temps de séchage sont faibles.

La présente invention a donc pour objet une composition cosmétique contenant, dans un milieu cosmétiquement acceptable, une dispersion aqueuse de particules insolubles de polymère et une émulsion de silicone α,ω-disilanol non aminée.

Les compositions selon l'invention présentent, outre les avantages précités, une bonne résistance à l'humidité, une bonne élimination au shampooing et au brossage et une bonne vitesse de séchage.

Mais d'autres caractéristiques, aspects ou avantages de l'invention apparaîtront encore plus complètement à la lecture de la description détaillée qui va suivre et des exemples concrets mais nullement limitatifs destinés à l'illustrer.

Les dispersions aqueuses de particules insolubles de polymère non ionique ou ionique utilisables selon l'invention sont généralement obtenues par polymérisation ou copolymérisation en suspension ou en émulsion de monomères selon les procédés bien connus de l'état de la technique (de telles dispersions sont aussi connues sous le nom de "latex"). On peut également obtenir des dispersions aqueuses de polymères en solubilisant ledit polymère dans un solvant organique miscible à l'eau, puis on ajoute de l'eau et enfin on évapore le solvant organique. Ce type de préparation est par exemple décrit dans la demande française n° 2 697 160.

Le diamètre moyen des particules insolubles de polymère est généralement inférieur à 500 nm et de préférence inférieur à 250 nm. La température de transition vitreuse du polymère est généralement comprise entre - 30°C et 150°C et de préférence entre 10 et 90°C.

Généralement, les dispersions contiennent au moins 0,5% de tensioactif permettant la mise en dispersion et le maintien en dispersion du polymère insoluble. Selon l'invention, on peut utiliser tout type de tensioactif, mais de préférence un tensioactif non ionique.

Le polymère de la dispersion aqueuse comprend au moins un monomère choisi par exemple parmi le styrène, le butadiène, l'éthylène, le tétrafluoroéthylène, le propylène, le vinyl toluène, le vinyl propionate, l'alcool vinylique, l'acrylonitrile, le chloroprène, le chlorure de vinyle, l'acétate de vinyle, les uréthannes, l'isoprène, les polyols, les diisocyanates, les triisocyanates, l'isobuténe, les éthers vinyliques, la vinylpyrrolidone, le vinylimidazole, les (méth)acrylate de triméthylammonioéthyle, les acides acrylique ou méthacrylique, maléïque, crotonique ou itaconique, leurs esters ou leurs amides et leurs mélanges.

Les polymères non ioniques dans les dispersions aqueuses utilisables selon la présente invention sont par exemple choisis parmi les composés suivants :
- les homopolymères d'acétate de vinyle tels que le produit proposé sous le nom de APPRETAN EM par la société HOECHST ou le produit proposé sous le nom de RHODOPAS A 012 par la société RHONE POULENC ;
- les copolymères d'acétate de vinyle et d'ester acrylique tels que le produit proposé sous le nom de RHODOPAS AD 310 de RHONE POULENC ;
- les copolymères d'acétate de vinyle et d'éthylène tels que le produit proposé sous le nom de APPRETAN TV par la société HOECHST ;
- les copolymères d'acétate de vinyle et d'ester maléïque par exemple de maléate de dibutyle tels que le produit proposé sous le nom de APPRETAN MB EXTRA par la société HOECHST ;
- les homopolymères de chlorure de vinyle tels que les produits proposés sous les noms de GEON 460X45, GEON 460X46 et GEON 577 par la société GOODRICH ;
- les cires de polyéthylène tels que les produits proposés sous les dénominations AQUACER 513 et AQUACER 533 par la société BYK CERA ;
- les cires de polyéthylène/polytétrafluoroéthylène tels que les produits proposés sous les dénominations DREWAX D-3750 par la société DREW AMEROID et WAX DISPERSION WD-1077 par la société R.T. NEWEY ;
- les copolymères de polyéthylène et d'anhydride maléïque ;
- les homopolymères d'acrylates d'alkyle et les homopolymères de méthacrylates d'alkyle tels que le produit proposé sous la dénomination MICROPEARL RQ 750 par la société MATSUMOTO ou le produit proposé sous la dénomination LUHYDRAN A 848 S par la société BASF ;
- les copolymères d'esters acryliques tels que par exemple les copolymères d'acrylates d'alkyle et de méthacrylates d'alkyles tels que les produits proposés par la société ROHM&HAAS sous les dénominations PRIMAL ACZ 61 k et EUDRAGIT NE 30 D, par la société BASF sous les dénominations ACRONAL 601, LUHYDRAW LR 8833 ou 8845, par la société HOECHST sous les dénominations APPRETAN N 9213 ou N9212;
- les copolymères d'acrylonitrile et d'un monomère non ionique choisi par exemple parmi le butadiène et les (méth)acrylates d'alkyle ; on peut citer les produits proposés sous les dénominations NIPOL LX 531 B par la société NIPPON ZEON ou ceux proposés sous la dénomination CJ 0601 B par la société ROHM & HAAS
- les homopolymères de styrène tels que le produit RHODOPAS 5051 proposé par la société RHONE POULENC ;
- les copolymères de styrène et de (méth)acrylate d'alkyle tels que les produits MOWILITH LDM 6911, MOWILITH DM 611 et MOWILITH LDM 6070 proposés par la société HOECHST, les produits RHODOPAS SD 215 et RHODOPAS DS 910 proposés par la société RHONE POULENC, le produit URAMUL SC 70 proposé par la société DSM ;
- les copolymères de styrène, de méthacrylate d'alkyle et d'acrylate d'alkyle tels que le produit DAITISOL SPA proposé par la société WACKHERR ;
- les copolymères de styrène et de butadiène tels que les produits RHODOPAS SB 153 et RHODOPAS SB 012 proposés par la société RHONE POULENC ;
- les copolymères de styrène, de butadiène et de vinylpyridine tels que les produits GOODRITE SB VINYLPYRIDINE 2528X10 et GOODRITE SB VINYLPYRIDINE 2508 proposés par la société GOODRICH ;
- les copolymères de styrène et de vinylpyrrolidone tels que les produits ANTARA 450 et CLOUD 285 proposés par la société ISP ;
- les polyuréthannes tels que les produits proposés sous les dénominations ACRYSOL RM 1020 ou ACRYSOL RM 2020 par la société ROHM & HAAS, les produits URAFLEX XP 401 UZ, URAFLEX XP 402 UZ par la société DSM RESINS ;
- les copolymères d'acrylate d'alkyle et d'uréthanne tels que le produit 8538-33 par la société NATIONAL STARCH ;
- les polyamides tels que le produit ESTAPOR LO 11 proposé par la société RHONE POULENC.

Les dispersions de particules insolubles de polymère cationique comprennent par exemple les polymères suivants :
- les copolymères d'acrylamide et de (méth)acrylate de triméthylammonioéthyle ;
- les copolymères de méthacrylate d'alkyle, d'acrylate d'alkyle et de (méth)acrylate de triméthylammonioéthyle tel que le produit EUDRAGIT RL 30 D proposé par la société ROHM PHARMA.

Les dispersions aqueuses de particules insolubles de polymère particulièrement préférées dans le cadre de l'invention sont les dispersions aqueuses de particules insolubles de polymères anioniques.

Selon l'invention, on peut par exemple utiliser une dispersion aqueuse comprenant un copolymère formé d'un acrylate d'alkyle, de méthacrylate d'alkyle et d'un ou plusieurs acides carboxyliques éthyléniques ayant de 3 à 5 atomes de carbone, les radicaux alkyle ayant de 1 à 5 atomes de carbone.

L'acrylate d'alkyle est de préférence choisi parmi l'acrylate de méthyle, l'acrylate d'éthyle, l'acrylate de propyle et l'acrylate de butyle. L'acrylate d'éthyle est particulièrement préféré.
La concentration en acrylate d'alkyle est de préférence comprise entre 40 et 70% en poids et plus particulièrement entre 50 et 60% en poids par rapport au poids total du copolymère.

Le méthacrylate d'alkyle est de préférence choisi parmi le méthacrylate de méthyle, le méthacrylate d'éthyle, le méthacrylate de propyle et le méthacrylate de butyle. Le méthacrylate de méthyle est particulièrement préféré.
La concentration en méthacrylate d'alkyle est de préférence comprise entre 30 et 50% en poids et plus particulièrement entre 30 et 40% en poids par rapport au poids total du copolymère.

Les acides carboxyliques éthyléniques préférés sont l'acide acrylique, l'acide méthacrylique, l'acide crotonique, l'acide itaconique ou leurs mélanges. L'acide acrylique et l'acide méthacrylique sont particulièrement préférés. Selon l'invention, il est possible de mettre en oeuvre des sels de ces acides carboxyliques.

La concentration en acides carboxyliques éthyléniques, ou en leurs sels, est de préférence comprise entre 5 et 15% en poids et plus particulièrement entre 8 et 12% en poids par rapport au poids total du copolymère.

Dans un mode de réalisation particulièrement préféré de l'invention, l'acide acrylique est utilisé avec l'acide méthacrylique, chacun dans une concentration comprise entre 2 et 10% en poids, le total de ces deux acides n'excédant pas 15% en poids du poids total du copolymère.

Le copolymère peut également contenir des faibles quantités, c'est à dire moins de 10%, de préférence moins de 5% et plus particulièrement moins de 2%, d'un monomère polymérisable autre que ceux mentionnés ci-avant.

Selon un mode particulièrement préféré de mise en oeuvre de l'invention, on utilise un copolymère comprenant de 50 à 60% en poids d'acrylate d'éthyle, de 30 à 40% en poids de méthacrylate de méthyle, de 2 à 10% en poids d'acide acrylique, de 2 à 10% en poids d'acide méthacrylique, la concentration totale d'acide acrylique et méthacrylique n'excédant pas 15% en poids par rapport au poids total du copolymère acrylique.

Un tel copolymère est par exemple décrit dans la demande de brevet EP-A-590604 qui est ici incluse ici à titre de référence.

Une dispersion aqueuse du copolymère acrylique décrit ci-dessus comprenant 25% en poids d'un copolymère acrylate d'éthyle / méthacrylate de méthyle/ acide méthacrylique/ acide acrylique est vendu notamment sous la dénomination commerciale AMERHOLD DR-25 par la société AMERCHOL.

Selon l'invention, on peut également utiliser une dispersion aqueuse de copolymères méthacrylate d'hydroxyéthyle / méthacrylate de méthyle / acide méthacrylique / acrylate de butyle tels que par exemple le produit commercialisé par la société SEPPIC sous la dénomination ACUDYNE 255.

Selon l'invention, on peut également utiliser une dispersion aqueuse de copolymères acrylate d'éthyle */* acide méthacrylique / acrylate t- butyle tels que par exemple le produit commercialisé par la société BASF sous la dénomination LUVIMER LOW VOC.

Selon l'invention, on peut également utiliser une dispersion aqueuse de copolymères méthacrylate de méthyle / acide acrylique */* acrylate de butyle tels que par exemple le produit commercialisé par la société NATIONAL STARCH sous la dénomination BALANCE 055.

La concentration en poids des particules de polymère insoluble dans les compositions selon l'invention est de préférence comprise entre 1 et 35% par rapport au poids total de la composition, de préférence entre 5 et 20% en poids.

Les émulsions de silicones α,ω-disilanols non aminée utilisables dans le cadre de la présente invention peuvent être choisis parmi toutes celles déjà connues en soi.

Selon l'invention, on désigne par silicone α,ω-disilanol non aminée toute silicone ne comportant pas au moins une amine primaire, secondaire, tertiaire ou un groupement ammonium quaternaire.

La taille moyenne des particules de silicone dans l'émulsion est de préférence comprise entre 1 nm et 10 microns et plus particulièrement entre 10 nm et 1 micron.

Les émulsions utilisables selon l'invention peuvent être des microémulsions, c'est à dire des émulsions thermodynamiquement stables.

Les émulsions sont généralement aqueuses et contiennent en plus de l'eau et de la silicone α,ω-disilanol non aminée un ou plusieurs tensioactifs. Ces tensioactifs peuvent être de tous type, et plus particulièrement de type non ionique ou cationique.

Ainsi, selon la présente invention, il est possible d'utiliser toute silicone α,ω-disilanol connue en soi, qu'il s'agisse d'une huile, d'une résine ou bien encore d'une gomme de silicone. Les silicones sont des polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane), des radicaux hydrocarbonés éventuellement substitués étant directement liés par l'intermédiaire d'un atome de carbone sur lesdits atomes de silicium. Les radicaux hydrocarbonés les plus courants sont les radicaux alkyles et en particulier méthyle, les radicaux fluoroalkyles, les radicaux aryles et en particulier phényle, et les radicaux alcényles et en particulier vinyle; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropyiène, les radicaux hydroxyles ou hydroxyalkyles, les radicaux acyloxy ou acyloxyalkyles, des groupements anioniques tels que carboxylates, thioglycolates, sulfosuccinates, thiosulfates, phosphates et sulfates, cette liste n'étant bien entendu nullement limitative (silicones dites "organomodifiées"). Le poids moléculaire moyen en nombre des silicones utilisables selon l'invention peut varier entre 100 et plusieurs millions, de préférence entre 1000 et 1 000 000. Selon la présente invention, on peut bien entendu soit utiliser une seule et même silicone soit, mettre en oeuvre plusieurs silicones différentes.

Ces silicones peuvent être réticulées.

A titre d'exemples de silicones utilisables dans les compositions selon l'invention, on peut notamment citer les polydialkylsiloxanes α,ω-disilanols, les polyalkylarylsiloxanes α,ω-disilanols et les polydiaryldialkylsiloxanes α,ω-disilanols.

Les groupements alkyles ont de préférence de 1 à 4 atomes de carbone et les groupements aryles sont de préférence phényle.

Selon un mode de réalisation particulièrement préféré de la présente invention, les silicones utilisées sont choisies parmi les polydiméthylsiloxanes α,ω-disilanols.

De tels produits sont par exemple l'émulsion aqueuse non ionique de polydiméthylsiloxanes α,ω-disilanols commercialisée sous la dénomination SILTECH E-2170 par la société SILTECH.

La ou les silicones sont présentes dans les compositions conformes à l'invention dans des proportions généralement comprises entre 0,05 à 10 % en poids, de préférence de 0,1 à 3 % en poids, par rapport au poids total de la composition.

Le milieu continu cosmétiquement acceptable servant de support aux compositions selon l'invention, est aqueux ou hydroalcoolique et de préférence constitué par de l'eau ou un mélange d'eau et de solvants cosmétiquement acceptables tels que des monoalcools, des polyalcools et des éthers de glycol, qui peuvent être utilisés seuls ou en mélange. Encore plus préférentiellement, ledit support est essentiellement constitué d'eau.

Le pH des compositions selon l'invention est généralement compris entre 2 et 9, et en particulier entre 3 et 8. Il peut être ajusté à la valeur désirée au moyen d'agents alcalinisants ou acidifiants habituellement utilisés en cosmétique pour ce type d'application.

Lorsque la composition selon l'invention est pressurisée sous forme d'aérosol, l'aérosol comprend la composition décrite ci-dessus, appelée jus, et au moins un agent propulseur qui peut être choisi parmi les hydrocarbures volatils tels que le n-butane, le propane, l'isobutane, le pentane, les hydrocarbures chlorés et/ou fluorés et leurs mélanges. On peut également utiliser en tant qu'agent propulseur le gaz carbonique, le protoxyde d'azote, le diméthyléther, l'azote, l'air comprimé et leurs mélanges.

Dans un tel système, la concentration en propulseur(s) est généralement comprise entre 5 et 90% et de préférence entre 10 et 50% en poids par rapport au poids total de la composition pressurisée et plus particulièrement entre 15 et 35% en poids.

Selon ce mode préféré de réalisation de l'invention, la concentration en particules de polymère est d'au moins 3 % en poids par rapport au poids de la composition pressurisée (jus + propulseur), et encore plus préférentiellement comprise entre 5 et 35% en poids.

Les compositions selon l'invention ( à l'état pressurisé ou non) peuvent encore contenir des agents tensioactifs, des agents conservateurs, des séquestrants, des adoucissants, des parfums, des colorants, des agents modificateurs de viscosité, des agents modificateurs de mousse, des agents anti-mousse, des agents nacrants, des agents hydratants, des agents antipelliculaires, des agents antiséborrhéiques, des filtres solaires, des céramides, des protéines, des vitamines, des plastifiants, des hydroxyacides, des électrolytes, des huiles et des cires naturelles ou synthétiques, des alcools gras, des esters d'alcools polyhydriques, des mono-, di- ou triglycérides, des polymères hydrosolubles ou des mélanges de ces différents composés..

Bien entendu, l'homme de l'art veillera à choisir le ou les éventuels composés à ajouter à la composition selon l'invention de manière telle que les propriétés avantageuses attachées intrinsèquement à la composition conforme à l'invention ne soient pas, ou substantiellement pas, altérées par l'addition envisagée.

Les compositions selon l'invention sont par exemple des compositions capillaires rincées ou non rincées et de préférence non rincées.

Elles sont plus particulièrement des lotions de mise en plis, des lotions pour le brushing, des compositions de fixation (laques) et de coiffage. Les lotions sont conditionnées sous diverses formes notamment dans des vaporisateurs, des flacons pompe ou dans des récipients aérosols afin d'assurer une application de la composition sous forme vaporisée.

L'invention a encore pour objet un procédé de traitement cosmétique des matières kératiniques, telles que les cheveux, caractérisé en ce qu'il consiste à appliquer sur les matières kératiniques ,en particulier par pulvérisation ou vaporisation, une composition cosmétique telle que définie précédemment, puis à effectuer éventuellement un rinçage à l'eau, après un éventuel temps de pose.

### EXEMPLE 1

On a préparé une composition A selon l'invention et on l'a comparée à trois compositions B, C et D non conformes à l'invention. Les quatre compositions sont conditionnées en flacon-pompe.

Un panel de testeurs a évalué le démêlage, la douceur et le toucher des cheveux après pulvérisation de 1 g de chacune de ces composition sur des mèches de cheveux naturels de 5 g.

La notation va de 0 (très mauvais) à 5 (excellent).

Les résultats sont rassemblés dans le tableau ci-dessous ( MA signifie matière active):

| En gMA | **A (Invention)** | **B (comparatif)** | **C (comparatif)** | **D (comparatif)** |
|---|---|---|---|---|
| **AMERHOLD DR 25**^{**(1)**} | 10 | 10 | 10 | 10 |
| **SIL TECH E-2170**^{**(2)**} | 1 | ― | ― | ― |
| **Q2-5220**^{**(3)**} | ― | 1 | ― | ― |
| **L7230** ^{**(4)**} | ― | ― | 1 | ― |
| **Phtalate d'éthyle** | 1,5 | 1,5 | 1,5 | 1,5 |
| **Eau qsp** | 100 | 100 | 100 | 100 |
| **Démêlage** | 3 | 2 | 0 | 3 |
| **Douceur** | 3 | 1,5 | 1 | 2 |
| Toucher | 3 | 1,5 | 1 | 2 |

| | | | | |
|---|---|---|---|---|
| (1) : AMERHOLD DR 25 de AMERCHOL : copolymère acrylate d'éthyle / méthacrylate de méthyle / acide méthacrylique / acide acrylique en dispersion aqueuse comprenant 25% en poids du copolymère. | | | | |
| (2) SILTECH E-2170 de SILTECH : Emulsion aqueuse non ionique à 60% en poids de polydiméthyl siloxane α,ω-disilanols (diméthiconol). | | | | |
| (3) Q2-5220 de DOW CORNING : Polydiméthylsiloxane polyoxyéthyléné et polyoxypropyléné (diméthicone copolyol) | | | | |
| (4) L7270 de OSI : Polydiméthylsiloxane polyoxyéthyléné et polyoxypropyléné (diméthicone copolyol) | | | | |

L'émulsion aqueuse de polydiméthyl siloxane α,ω-disilanols (A) permet d'améliorer la douceur et le toucher des cheveux alors que le diméthicone copolyol (B et C) diminue ces deux propriétés.

### EXEMPLE 2

On a préparé deux compositions A et B selon l'invention et on les a comparées à deux compositions C et D non conformes à l'invention. Les quatre compositions sont conditionnées en flacon-pompe.

Un panel de testeurs a évalué le démêlage, la douceur et le toucher des cheveux après pulvérisation de 1 g de chacune de ces composition sur des mèches de cheveux naturels de 5 g.

Les résultats sont rassemblés dans le tableau ci-dessous :

| En gMA | **A (Invention)** | **B (Invention)** | **C (comparatif)** | **D (comparatif)** |
|---|---|---|---|---|
| **ACUDYNE 255**^{**(1)**} | 10 | 10 | 10 | 10 |
| **SILTECH E-2170**^{**(2)**} | 1 | ― | ― | ― |
| **Q2-5220**^{**(3)**} | ― | ― | 1 | ― |
| **TP511 A**^{**(4)**} | ― | 1 | ― | ― |
| **Phtalate d'éthyle** | 3 | 3 | 3 | 3 |
| **Eau qsp** | 100 | 100 | 100 | 100 |
| **Démêlage** | 4 | 4,5 | 2,5 | 4 |
| Douceur | 3 | 3,5 | 2 | 2,5 |
| Toucher | 3 | 3 | 2 | 2,5 |

| | | | | |
|---|---|---|---|---|
| (1) ACUDYNE 255 de SEPPIC : copolymère méthacrylate d'hydroxyéthyle / méthacrylate de méthyle / acide méthacrylique */* acrylate de butyle en dispersion aqueuse comprenant environ 40% en poids du copolymère. | | | | |
| (2) SILTECH E-2170 de SILTECH : Emulsion aqueuse non ionique à 60% en poids de polydiméthyl siloxane α,ω-disilanols (diméthiconol). | | | | |
| (3) Q2-5220 de DOW CORNING : Polydiméthylsiloxane polyoxyéthyléné et polyoxypropyléné (diméthicone copolyol) | | | | |
| (4) TP511 A de OSI : Emulsion aqueuse anionique à 35% en poids de polydiméthyl siloxane α,ω-disilanols (diméthiconol) réticulé. | | | | |

Les émulsions aqueuses de polydiméthyl siloxane α,ω-disilanols (A et B) permettent d'améliorer le démêlage, la douceur et le toucher des cheveux alors que le diméthicone copolyol (B) diminue ces trois propriétés.

### EXEMPLE 3

On a préparé une composition A selon l'invention et on l'a comparée à deux compositions B et C non conformes à l'invention. Les trois compositions ont été pressurisées en aérosol.

Un panel de testeurs a évalué le démêlage, la douceur et le toucher des cheveux après pulvérisation de 2 g de chaque composition sur des mèches de cheveux naturels de 5 g.

Les résultats sont rassemblés dans le tableau ci-dessous ( MA signifie matière active):

| En gMA | **A (Invention)** | **B (comparatif)** | **C (comparatif)** |
|---|---|---|---|
| **AMERHOLD DR 25**^{**(1)**} | 15,4 | 15,4 | 15,4 |
| **SIL TECH E-2170**^{**(2)**} | 1,5 | ― | ― |
| **Q2-5220** ^{**(3)**} | ― | 1,5 | ― |
| **Phtalate d'éthyle** | 2,3 | 2,3 | 2,3 |
| **Eau qsp** | 100 | 100 | 100 |
| **Démêlage** | 2 | 1 | 2 |
| **Douceur** | 2,5 | 1,5 | 2 |
| **Toucher** | 2,5 | 1,5 | 2 |

| | | | |
|---|---|---|---|
| (1) : AMERHOLD DR 25 de AMERCHOL : copolymère acrylate d'éthyle / méthacrylate de méthyle / acide méthacrylique / acide acrylique en dispersion aqueuse comprenant 25% en poids du copolymère. | | | |
| (2) SILTECH E-2170 de SILTECH : Emulsion aqueuse non ionique à 60% en poids de polydiméthyl siloxane α,ω-disilanols (diméthiconol). | | | |
| (3) Q2-5220 de DOW CORNING : Polydiméthylsiloxane polyoxyéthyléné et polyoxypropyléné (diméthicone copolyol) | | | |

Le schéma de pressurisation était le suivant :
- diméthyléther (propulseur) 35 g
- Composition ci-dessus (jus) 65 g

L'émulsion aqueuse de polydiméthyl siloxane α,ω-disilanols permet d'améliorer la douceur et le toucher des cheveux alors que le diméthicone copolyol diminue ces deux propriétés.

## Revendications

1. Composition cosmétique; **caractérisée par le fait qu'**elle comprend, dans un milieu cosmétiquement acceptable, une dispersion aqueuse de particules insolubles de polymère et une émulsion de silicone α,ω-disilanols non aminée ; la ou les silicones α,ω-disilanol non aminée étant présentes à raison de 0,05 à 10 % en poids par rapport au poids total de la composition.

2. Composition selon la revendication 1, **caractérisée par le fait que** ladite dispersion aqueuse résulte de la polymérisation ou de la copolymérisation de monomères choisis parmi le styrène, le butadiène, l'éthylène, le propylène, le vinyl toluène, le vinyl propionate, l'alcool vinylique, l'acrylonitrile, le chloroprène, l'acétate de vinyle, les uréthannes, l'isoprène, l'isobutène, l'éther vinylique, la vinylpyrrolidone, le vinylimidazole et les acides acrylique ou méthacrylique, maléïque, crotonique ou itaconique, leurs esters ou leurs amides.

3. Composition selon l'une quelconques des revendications 1 ou 2, **caractérisée par le fait que** le polymère de la dispersion aqueuse est choisi parmi les copolymères d'acrylate d'alkyle, de méthacrylate d'alkyle et d'un ou plusieurs acides carboxyliques éthyléniques ou leurs sels ayant de 3 à 5 atomes de carbone, les radicaux alkyle ayant de 1 à 5 atomes de carbone.

4. Composition selon l'une quelconque des revendications précédente, **caractérisée en ce que** le polymère est choisi parmi les copolymères acrylate d'éthyle */* méthacrylate de méthyle */* acide méthacrylique */* acide acrylique.

5. Composition selon l'une quelconque des revendications 1 à 3, **caractérisée en ce que** le polymère de la dispersion aqueuse est choisi parmi les copolymères méthacrylate d'hydroxyéthyle */* méthacrylate de méthyle */* acide méthacrylique */* acrylate de butyle, les copolymères acrylate d'éthyle */* acide méthacrylique / acrylate t-butyle, les copolymères méthacrylate de méthyle / acide acrylique / acrylate de butyle.

6. Composition selon l'une quelconque des revendications précédentes, **caractérisée par le fait que** la concentration en poids des particules de polymère est comprise entre 1 et 35% par rapport au poids total de la composition.

7. Composition selon la revendication 1, **caractérisée par le fait que** la ou les silicones sont présentes à raison de 0,1 à 3 % en poids par rapport au poids total de la composition.

8. Composition selon l'une quelconque des revendications précédentes, **caractérisées par le fait que** la ou les silicones sont choisies parmi les polydialkylsiloxanes α,ω-disilanols, les polyalkylarylsiloxanes α,ω-disilanols et les polydiaryldialkylsiloxanes α,ω-disilanols.

9. Composition selon la revendication 8, **caractérisées par le fait que** la ou les silicones sont choisies parmi les polydiméthylsiloxanes α,ω-disilanols.

10. Composition selon l'une quelconques des revendications précédentes, **caractérisée par le fait que** les compositions sont des lotions de mise en plis, des lotions pour le brushing.

11. Composition pressurisée en aérosol, **caractérisée par le fait qu'**elle comprend une composition telle que définie à l'une quelconque des revendications 1 à 9, et au moins un agent propulseur.

12. Composition selon la revendication 11, **caractérisée par le fait que** l'agent propulseur est choisi parmi les hydrocarbures volatils tels que le n-butane, le propane, l'isobutane, le pentane, les hydrocarbures chlorés et/ou fluorés, le gaz carbonique, le protoxyde d'azote, le diméthyléther, l'azote, l'air comprimé et leurs mélanges.

13. Composition selon l'une quelconque des revendications 11 ou 12, **caractérisée par le fait que** la concentration en poids des particules de polymère est d'au moins 3 % en poids et de préférence comprise entre 5 et 35% par rapport au poids total de la composition pressurisée.

14. Composition selon l'une quelconque des revendications 11 à 13, **caractérisée par** le fait l'agent propulseur est présent dans des concentrations comprises entre 5 et 90% en poids par rapport au poids total de la composition pressurisée.

15. Composition selon la revendication 14, **caractérisée par** le fait l'agent propulseur est présent dans des concentrations comprises entre 10 et 50% en poids par rapport au poids total de la composition pressurisée.

16. Procédé de traitement cosmétique des matières kératiniques, telles que les cheveux, **caractérisé en ce qu'**il consiste à appliquer sur les matières kératiniques, une composition cosmétique telle que définie dans l'une quelconque des revendications 1 à 15, puis à effectuer éventuellement un rinçage à l'eau, après un éventuel temps de pose.

## Patentansprüche

1. Kosmetische Zusammensetzung, **dadurch gekennzeichnet, daß** sie in einem kosmetisch akzeptablen Medium eine wäßrige Dispersion von unlöslichen Polymerpartikeln und eine Emulsion eines nicht aminierten α,ω-Disilanol-silicons enthält, wobei das nicht aminierte α,ω-Disilanol-silicon oder die nicht aminierten α,ω-Disilanol-silicone in einem Mengenanteil von 0,05 bis 10 Gew.-%, bezogen auf das Gesamtgewicht der Zusammensetzung, vorliegen.

2. Zusammensetzung nach Anspruch 1, **dadurch gekennzeichnet, daß** die wäßrige Dispersion bei der Polymerisation oder Copolymerisation von Monomeren entsteht, die unter Styrol, Butadien, Ethylen, Propylen, Vinyltoluol, Vinylpropionat, Vinylalkohol, Acrylnitril, Chloropren, Vinylacetat, Urethanen, Isopren, Isobuten, Vinylether, Vinylpyrrolidon, Vinylimidazol und Acrylsäure, Methacrylsäure, Maleinsäure, Crotonsäure oder Itaconsäure und deren Estern oder deren Amiden ausgewählt sind.

3. Zusammensetzung nach einem der Ansprüche 1 oder 2, **dadurch gekennzeichnet, daß** das Polymer der wäßrigen Dispersion unter den Copolymeren von Alkylacrylat, Alkylmethacrylat und einer oder mehrerer Carbonsäuren mit ethylenischer Doppelbindung oder ihrer Salze mit 3 bis 5 Kohlenstoffatomen ausgewählt ist, wobei die Alkylgruppen 1 bis 5 Kohlenstoffatome aufweisen.

4. Zusammensetzung nach einem der vorhergehenden Ansprüche, **dadurch gekennzeichnet, daß** das Polymer unter den Ethylacrylat/Methylmethacrylat/ Methacrylsäure/Acrylsäure-Copolymeren ausgewählt ist.

5. Zusammensetzung nach einem der Ansprüche 1 bis 3, **dadurch gekennzeichnet, daß** das Polymer der wäßrigen Dispersion unter den Hydroxyethylmethacrylat/Methylmethacrylat/Methacrylsäure/Butylacrylat-Copolymeren, Ethylacrylat/ Methacrylsäure/t-Butylacrylat-Copolymeren und Methylmethacrylat/ Acrylsäure/Butylacrylat-Copolymeren ausgewählt ist.

6. Zusammensetzung nach einem der vorhergehenden Ansprüche, **dadurch gekennzeichnet, daß** die Konzentration der Polymerpartikel im Bereich von 1 bis 35 Gew.-%, bezogen auf das Gesamtgewicht der Zusammensetzung, liegt.

7. Zusammensetzung nach Anspruch 1, **dadurch gekennzeichnet, daß** das Silicon oder die Silicone in Mengenanteilen von 0,1 bis 3 Gew.-%, bezogen auf das Gesamtgewicht der Zusammensetzung, vorliegen.

8. Zusammensetzung nach einem der vorhergehenden Ansprüche, **dadurch gekennzeichnet, daß** das Silicon oder die Silicone unter den α,ω-Disilanol-polydialkylsiloxanen, α,ω-Disilanol-polyalkylarylsiloxanen und α,ω-Disilanol-polydiaryldialkylsiloxanen ausgewählt sind.

9. Zusammensetzung nach Anspruch 8, **dadurch gekennzeichnet, daß** das Silicon oder die Silicone unter den α,ω-Disilanol-poly dimethylsiloxanen ausgewählt sind.

10. Zusammensetzung nach einem der vorhergehenden Ansprüche, **dadurch gekennzeichnet, daß** es sich bei den Zusammensetzungen um Lotionen für Wasserwellen und Lotionen zum Fönen handelt.

11. Als Aerosol unter Druck gesetzte Zusammensetzung, **dadurch gekennzeichnet, daß** sie eine Zusammensetzung nach einem der Ansprüche 1 bis 9 und mindestens ein Treibmittel enthält.

12. Zusammensetzung nach Anspruch 11, **dadurch gekennzeichnet, daß** das Treibmittel unter den flüchtigen Kohlenwasserstoffen, wie n-Butan, Propan, Isobutan, Pentan, chlorierten und/oder fluorierten Kohlenwasserstoffen, Kohlendioxid, Distickstoffoxid, Dimethylether, Stickstoff, Druckluft und deren Gemischen ausgewählt ist.

13. Zusammensetzung nach einem der Ansprüche 11 oder 12, **dadurch gekennzeichnet, daß** die Konzentration der Polymerpartikel mindestens 3 Gew.-% beträgt und vorzugsweise im Bereich von 5 bis 35 Gew.-%, bezogen auf das Gesamtgewicht der unter Druck gesetzten Zusammensetzung, vorliegt.

14. Zusammensetzung nach einem der Ansprüche 11 bis 13, **dadurch gekennzeichnet, daß** das Treibmittel in Konzentrationen von 5 bis 90 Gew.-%, bezogen auf das Gesamtgewicht der unter Druck gesetzten Zusammensetzung, vorliegt.

15. Zusammensetzung nach Anspruch 14, **dadurch gekennzeichnet, daß** das Treibmittel in Konzentrationen von 10 bis 50 Gew.-%, bezogen auf das Gesamtgewicht der unter Druck gesetzten Zusammensetzung, vorliegt.

16. Verfahren zur kosmetischen Behandlung von Keratinsubstanzen, wie dem Haar, **dadurch gekennzeichnet, daß** es darin besteht, auf die Keratinsubstanzen eine kosmetische Zusammensetzung nach einem der Ansprüche 1 bis 15 aufzubringen und dann gegebenenfalls nach einer Einwirkzeit gegebenenfalls mit Wasser zu spülen.

## Claims

1. Cosmetic composition, **characterized in that** it comprises, in a cosmetically acceptable medium, an aqueous dispersion of insoluble particles of polymer and a non-aminated silicone α,ω-disilanol emulsion, the non-aminated silicone α,ω-disilanol(s) being present in the proportion of 0.05 to 10% by weight with respect to the total weight of the composition.

2. Composition according to Claim 1, **characterized in that** the said aqueous dispersion results from the polymerization or from the copolymerization of monomers chosen from styrene, butadiene, ethylene, propylene, vinyltoluene, vinyl propionate, vinyl alcohol, acrylonitrile, chloroprene, vinyl acetate, urethanes, isoprene, isobutene, vinyl ether, vinylpyrrolidone, vinylimidazole and acrylic or methacrylic, maleic, crotonic or itaconic acids, their esters or their amides.

3. Composition according to either one of Claims 1 and 2, **characterized in that** the polymer of the aqueous dispersion is chosen from copolymers of alkyl acrylate, of alkyl methacrylate and of one or more ethylenic carboxylic acids or their salts having from 3 to 5 carbon atoms, the alkyl radicals having from 1 to 5 carbon atoms.

4. Composition according to any one of the preceding claims, **characterized in that** the polymer is chosen from ethyl acrylate/methyl methacrylate/methacrylic acid/acrylic acid copolymers.

5. Composition according to any one of Claims 1 to 3, **characterized in that** the polymer of the aqueous dispersion is chosen from hydroxyethyl methacrylate/methyl methacrylate/methacrylic acid/butyl acrylate copolymers, ethyl acrylate/methacrylic acid/t-butyl acrylate copolymers and methyl methacrylate/acrylic acid/butyl acrylate copolymers.

6. Composition according to any one of the preceding claims, **characterized in that** the concentration by weight of the polymer particles is between 1 and 35% with respect to the total weight of the composition.

7. Composition according to Claim 1, **characterized in that** the silicone or silicones are present in the proportion of 0.1 to 3% by weight with respect to the total weight of the composition.

8. Composition according to any one of the preceding claims, **characterized in that** the silicone or silicones are chosen from polydialkylsiloxane α,ω-disilanols, polyalkylarylsiloxane α,ω-disilanols and polydiaryldialkylsiloxane α,ω-disilanols.

9. Composition according to Claim 8, **characterized in that** the silicone or silicones are chosen from polydimethylsiloxane α,ω-disilanols.

10. Composition according to any one of the preceding claims, **characterized in that** the compositions are hairsetting lotions or blow-drying lotions.

11. Composition pressurized in an aerosol, **characterized in that** it comprises a composition as defined in any one of Claims 1 to 9 and at least one propellant.

12. Composition according to Claim 11, **characterized in that** the propellant is chosen from volatile hydrocarbons, such as n-butane, propane, isobutane, pentane or chlorinated and/or fluorinated hydrocarbons, carbon dioxide gas, nitrous oxide, dimethyl ether, nitrogen, compressed air and their mixtures.

13. Composition according to either one of Claims 11 and 12, **characterized in that** the concentration by weight of the polymer particles is at least 3% by weight and preferably between 5 and 35% with respect to the total weight of the pressurized composition.

14. Composition according to any one of Claims 11 to 13, **characterized in that** the propellant is present in concentrations of between 5 and 90% by weight with respect to the total weight of the pressurized composition.

15. Composition according to Claim 14, **characterized in that** the propellant is present in concentrations of between 10 and 50% by weight with respect to the total weight of the pressurized composition.

16. Process for the cosmetic treatment of keratinous substances, such as hair, **characterized in that** it consists in applying, to the keratinous substances, a cosmetic composition as defined in any one of Claims 1 to 15 and in then optionally rinsing with water, optionally after leaving for a period of time.
